Europäisches Patentamt

European Patent Office

Office européen des brevets

(11) Publication number: **0 327 918**
A1

## (12) EUROPEAN PATENT APPLICATION

(21) Application number: 89101575.2

(22) Date of filing: 30.01.89

(51) Int. Cl.⁴: **G01N 33/543** , **G01N 33/542** , **G01N 33/535**

(30) Priority: 28.01.88 JP 18908/88 U

(43) Date of publication of application:
16.08.89 Bulletin 89/33

(84) Designated Contracting States:
DE GB

(71) Applicant: KONICA CORPORATION
No. 26-2, Nishi-Shinjuku 1-chome
Shinjuku-ku Tokyo 163(JP)

(72) Inventor: Onishi, Akira
c/o Konica Corporation 1, Sakuramachi
Hino-shi Tokyo(JP)
Inventor: Ito, Tsukasa
c/o Konica Corporation 1, Sakuramachi
Hino-shi Tokyo(JP)

(74) Representative: Henkel, Feiler, Hänzel &
Partner
Möhlstrasse 37
D-8000 München 80(DE)

(54) Immunoassay element.

(57) An immunoassay element for assaying a target substance in a fluid sample by utilizing homogeneous competitive reaction of the target substance and a labelled substance which is a target substance or an analogue thereof labelled with a marker, with a substance which specifically reacts with both the target substance and the labelled substance. The immunoassay element comprises, in the order mentioned from the top to the bottom, the layers of a) a fibrous porous spreading layer; b) a non-fibrous porous reaction layer comprising the substance which specifically reacts with both the target substance and the labelled substance; and c) a non-porous layer comprising a macromolecular substance in which the labelled substance is incorporated. The labelled substance gives a signal which is changed when the labelled substance is bound to the substance which specifically reacts with both the target substance and the labelled substance.

EP 0 327 918 A1

# IMMUNOASSAY ELEMENT

## BACKGROUND OF THE INVENTION

### I. Field of the Invention

This invention relates to an immunoassay element. More particularly, this invention relates to an immunoassay element by which a target substance is assayed by utilizing homogeneous competitive reaction of the target substance and a labelled substance, with a substance which specifically reacts with both the target substance and the labelled substance.

### II. Description of the Related Art

A number of assay for detecting a substance contained in a biological fluid in an very small amount have been developed. Among these, the most sensitive assay is the immunoassay.

Immunoassay was first invented by Berson and Yallow in 1958. They succeeded in quantifying insulin in blood using bovine insulin labelled with radioactive iodine and anti-insulin antibody. Thereafter, radioimmunoassay has been widely used.

Various markers other than the radioactive markers have been developed. The markers other than the radioactive markers include enzymes, substrates of enzymes, coenzymes, enzyme inhibitors, bacteriophages, circulation reactant, complexes of metals or organic metals, organic prosthetic groups, chemiluminescent reactant and fluorescent molecules.

One of the important technical problems in immunoassay is so called B/F separation by which a bound substance and free substance are separated.

To solve the problem of B/F separation, various methods have been developed. One of these methods is the homogeneous immunoassay as disclosed in, for example, Japanese Patent Disclosure (Kokai) Nos. 146295/76, 2997/80, 211662/83, 20134/79, 104896/80 and 150681/82. In the homogeneous immunoassay, a target substance which is to be assayed and a labelled target substance are competitively reacted with a substance which specifically reacts with both the target substance and the labelled target substance. If the labelled target substance is bound to the substance which specifically reacts with both the target substance and the labelled target substance, a signal which indicates the binding of the labelled target substance is generated. By using such a labelled target substance, the B/F separation can be omitted.

In the field of clinical analysis, wet chemistry assay methods of such a homogeneous immunoassay are employed. However, in the wet homogeneous immunoassay, since a sample and various reagents are reacted in a relatively large amount of aqueous solution, and since a number of sampling and/or pipetting steps are involved, high level of skill and experience are required for the operator to obtain the reliable results, and so the reproducibility is also not good. In order to dissolve the problems in operation and to promote the sensitivity and reproducibility of the wet homogeneous immunoassay, measuring apparatuses in which the various operations are automated have been developed. However, complicated mechanism is required for the feeding of fluid and prevention of carrying over of the sample, so that the apparatuses are very expensive. Further, preparation of reagents, adjustment of the appratus and disposal of the sample fluid are troublesome. Still further, relatively large amount of sample is required and skillfulness is needed for the operation of the apparatus.

On the other hand, dry chemistry assay methods have been proposed by which the assay can be conducted by a simple operation. For example, in Japanese Patent Disclosure (Kokai) No. 103055/82, a monolayered element prepared by sequentially impregnating a filter paper or a fabric made of glass fibers with immunological reagents and coloring reagents. By this, a target substance may be assayed quickly with an inexpensive device. However, since the measurement is conducted on a rough surface of the filter paper or the like, high level of stray light and noise are generated, so that the precision and the reproducibility of the assay are not satisfactory.

A multilayered element utilizing the homogeneous immunoassay is disclosed in Japanese Patent Disclosure (Kokai) No. 18167/83. In this immunoassay element, the labelled substance and a substance (e.g., antibody) which specifically reacts with the target substance and the labelled substance are incorporated in a reaction layer made of a porous matrix. In most cases, the antibody which is contained in the reaction layer is prepared by immunizing an immunogen to the animal. Most of the low molecular substances are called haptens, and they have no immunogenecity by themselves. Therefore, the target substance, i.e., the hapten, is administered to the animals after bound to a carrier protein such as albumin via covalent bond. In general, the antibody prepared by immunizing such a hapten-carrier conjugate has a greater affinity to the labelled substance than to the target substance. Therefore, in measuring the hapten in a sample fluid, more sensitive assay can be attained by allowing the reaction between the hapten and the antibody prior to the reaction between the labelled substance and the antibody. However, if the antibody and the labelled substance are incorporated in the reaction layer as disclosed in the above-described Japanese Patent Disclosure, the reaction between the labelled substance and the antibody cannot be controlled satisfactorily.

On the other hand, in the immunoassay using a multilayered immunoassay element disclosed in Japanese Patent Disclosure No. 18167/83, a large amount of sample fluid as much as 25 - 200 $\mu$l is required. Since the sample fluid is used in such a large amount, immunological reagents are needed in correspondingly large amount. As a result, the element is expensive.

## SUMMARY OF THE INVENTION

Accordingly, the object of the present invention is to provide an immunoassay element by which a target substance in a sample fluid can be assayed with high sensitivity, high precision and high reproducibility using a small amount of sample fluid by simple operation.

This and other objects of the present invention may be accomplished by providing an immunoassay element for assaying a target substance in a fluid sample by utilizing homogeneous competitive reaction of the target substance and a labelled substance which is a target substance or an analogue thereof labelled with a marker, with a substance which specifically reacts with both the target substance and the labelled substance. The immunoassay element comprises, in the order mentioned from the top to the bottom, the layers of a) a fibrous porous spreading layer; b) a non-fibrous porous reaction layer comprising the substance which specifically reacts with both the target substance and the labelled substance; and c) a non-porous layer comprising a macromolecular substance in which the labelled substance is incorporated. The labelled substance gives a signal which is changed when the labelled substance is bound to the substance which specifically reacts with both the target substance and the labelled substance.

In the immunoassay element of the present invention, since the labelled substance is incorporated in a hydrophilic macromolecule forming a separate layer other than the reaction layer, the reaction between the labelled substance and the substance which specifically reacts both the target substance and the labelled substance may be deffered as desired under control. Therefore, the competitive reaction of the target substance and the labelled substance can be carried out so as to provide accurate measurement results. Thus, by using the immunoassay element of the present invention, the target substance in the sample fluid can be assayed with high sensitivity, high precision and high reproducibility using a small amount of sample fluid by simple operation.

## DETAILED DESCRIPTION OF THE PREFERRED EMBODIMENTS

As mentioned above, the multilayered immunoassay element of the present invention includes at least three layers of a) fibrous porous spreading layer, b) non-fibrous porous reaction layer, and c) non-porous layer, in the order mentioned from the top to the bottom of the element. Each of the layers will now be described in detail.

a) Fibrous Porous Developing Layer

The fibrous spreading layer serves for spreading the sample fluid so as to uniformly supply the sample fluid to the non-fibrous porous reaction layer under the spreading layer. The term "porous" herein means that the layer has communication pores through which the sample fluid can freely contact another layer. To attain the "porous" structure, the diameter of the communication holes may preferably be 1 - 300 $\mu$m. Examples of the material for forming the spreading layer may include pulp, cellulose powder and natural, synthetic and semisynthesized fibers of various plants, animals, minerals and artificial products such as cotton, hemp, silk, wool, chitin, chitosan, cellulose ester, viscose rayon, copper-ammonia rayon, polyamides (e.g., 6-Nylon, 6,6-Nylon and the like), polyesters (e.g., polyethylene terephthalate and the like), polyolefins (polypropyrene, vinylon and the like), glass and asbestos. These materials may be employed individually or in combination. Further, water-absorptive European paper, Japanese paper, filter paper and brush polymer, as well as the woven fabrics, non-woven fabrics and synthetic paper made from one or more of the above-described fibers may also be employed. The thickness of the spreading layer may preferably be 50 - 500 μm, more preferably 100 - 300 μm.

In order to eliminate the non-specific reaction in the immunological reaction, the spreading layer may contain a protein which does not participate or interfere the specific immunological reaction. Examples of such a protein include normal serum proteins of mammals, albumin and gelatin, as well as decomposition products thereof. The spreading layer per se is known in the art.

b) Non-fibrous Porous Reaction Layer

The term "non-fibrous" means the isotropic structures which are not fibrous. The term "porous" means the same meaning as described in a), although the preferred diameter of the communication holes is 1 - 100 um. Although any "non-fibrous" and "porous" material may be employed as the reaction layer, it is preferred that the reaction layer be constituted by a material in the form of powder. Preferred examples of the material in powder form may include diatomateous earth, titanium dioxide, barium sulfate, zinc oxide, lead oxide, microcrystalline cellulose, siliceous sand, glass, silica gel, crosslinked dextrane, crosslinked polyacrylamide, agarose, crosslinked agarose and various synthetic polymer such as polystyrene, as well as the self-binding particles consisting essentially of the compounds having the reactive groups such as oxiranyl group, glycidyl group, aziridinyl group, hydroxy group, amino group, carboxy group, thiol group, isocyanate group and vinyl sulfonyl group, which are described in detail in Japanese Patent Disclosure (Kokai) Nos. 101760/82 and 101761/82. These materials may be employed individually or in combination. The size of the particles constituting the powder may preferably be 1 - 100 μm.

In cases where the powder material does not have self-binding property, an appropriate adhesive or binder as hereinbelow described in more detail is used for forming the layer with the particles constituting the powder.

In the reaction layer, a substance which specifically reacts with both the target substance and the labelled substance is incorporated. The target substance is the substance in the sample fluid, which is to be detected or quantified, and the labelled substance is the target substance or an analogue thereof which is labelled with an appropriate marker. The labelled substance will be described in detail in the subsequent section.

Examples of the target substance may include polypeptides, proteins, conjugated proteins, carbohydrates, lipids, complex lipids, nucleic acids, hormones or hormone-like substances, vitamins, drugs and metabolites thereof, antibiotics and agricultural chemicals. It is necessary that it is possible to provide a substance such as an antibody which specifically reacts or binds the target substance. The target substance may preferably be a low molecular substance with a molecular weight of not more than 6000. Non-limiting specific examples of the target substance will now be enumerated.

[Hormones and Hormone-like Substances]

Follicle stimulating hormone (FSH), luteotropic hormone (LH), growth hormone (GH), thyroid stimulating hormone (TSH), adrenocorticotropic hormone (AcTH), melanin stimulating hormone (MHS), vasopressin, oxytocin, insulin, glucagon, angiotensin I, II, prolactin, secretin, dopamine, serotonin, somatostatin, thyroxine (T₄), triiodothyronine (T₃), gastrin, cortisol, aldosterone, catecholamine, estrogen, progesteron, testosterone, placental gonadotropin, placental lactogen, pituitary hormone releasing factors (TRH, FSH-RH, CRH, LH-RH and the like).

[Vitamins]

Biotin, thiamine, vitamin A, vitamin $B_2$, vitamin $B_6$, vitamin $B_{12}$, vitamin C, vitamin D, vitamin E, vitamin K and folic acid.

[Drugs and Metabolites Thereof]

Benzoyl ecgonine, cocaine, codeine, dextromethorphan, heroin, lysergic acid, morphine, quinidine, quinine, amycacin, gentamycin, kanamycin, neomycin, tobramycin, actinomycetin, karomycin, chloramphenicol, chloromycetin, chlorotetracycline, erythromycin, oxytetracycline, penicillin, cephalosporin, polymyxin B, terramycin, tetracycline, streptomycin, diphenylhydantoin, ethosuximide, phenobarbital, primidone, secobarbital, acetaminophenone, amitriptyline, carbamazepin, digoxin, disopyramide, lidocaine, methotrexate, N-acetylprocainamide, phenytoin, procainamide, propranolol, theophyllin, canabinol, antihistaminic agent, atropine, butyrophenone, caffeine, chloropromazine, barbiturate, amphetamine, catecholamine, epinephrine, griseofulvin, imipramine, L-DOPA, meperidine, meprobamate, methadone, narceine, nortriptyline, oxazepam, papaverine, prostaglandin and valproic acid, as well as the metabilites thereof.

[Agricultural Chemicals]

Halogenated biphenyl, phosphate esters and thiophosphates, as well as the metabolites thereof.

In the non-fibrous porous reaction layer, a substance which specifically reacts with both the target substance and the labelled substance is incorporated. This substance is hereinafter referred to as "target-binding substance" for simplicity. The target-binding substance may be, for example, an antibody, antigen, lectin or protein A depending on the target substance. It is preferred that the reaction between the target substance and the target-binding substance be an antigen-antibody reaction. The antibody which may be used as the target-binding substance may be obtained by a well-known conventional method. That is, the antibody may be produced by immunizing an animal with the target substance. In cases where the target substance has no immunogenecity, a conjugate of the target substance and a carrier protein such as albumin may be used for the immunization. Such a conjugate is well-known in the art. The antiserum or ascites collected from the immunized animal may be used as it is. Alternatively, the antibody may be used after purification by a well-known method such as sodium sulfate sedimentation method, ammonium sulfate sedimentation method, gel filtration method by using, for example, Sephadex gel (commercially available from Pharmacia), ion exchange cellulose chromatography method and electrophoresis method (the purification method is described in detail in, for example, Shunsuke Uda ed. "Immunochemistry", pp.74-84, published by Nakayama Shoten). Alternatively, a monoclonal antibody may also be employed. The monoclonal antibody specific to the target substance may be prepared by the well-known hybridoma technology. Using a monoclonal antibody is preferred in view of the high specificity. Monoclonal antibody in any class such as IgG, IgM and IgA, as well as a polyclonal antibody or antiserum may be used. Further, Fab, Fab' and F(ab')$_2$ fragments with antibody activity may also be used. These antibodies or fragments thereof may be used individually or in combination. In cases where the target substance is an antibody, the corresponding antigen may conveniently used as the target-binding substance.

The target-binding substance such as the above-described antibody should be incorporated in the reaction layer without losing the ability to bind to the target substance and the labelled substance. This may be achieved by, for example, incorporating a lyophilized mixture of the target-binding substance and normal serum proteins, albumin, gelatin and the like or a decomposition product thereof. Alternatively, the target-binding substance may be incorporated in the reaction layer by binding it to the above-mentioned material constituting the reaction layer or to a water-insoluble carrier ("Affinity Chromatography" (published by Kodansha Scientific, 1976), latex and the like via physical or chemical bond.

In the reaction layer, a substance which is necessary to detect the signal from the labelled substance may be incorporated. Particularly, in cases where the marker in the labelled substance is a prosthetic group or a coenzyme of an enzyme, the apoenzyme may be incorporated in the reaction layer. Preferred examples of the apoenzyme may include those described in Japanese Patent Disclosure (Kokai) Nos. 2997/80 and 103055/82, that is, the preferred examples of the apoenzyme may include, describing in the form of holoenzymes, glucose oxidase, glutathion reductase, lipoamide reductase, glutamate dehydrogenase, alcohol dehydrogenase, lactate dehydrogenase and the like. Among these, glucose oxidase is most preferred. In cases where the apoglucose oxidase is employed, it is preferred to co-use anti-glucose oxidase antibody for stabilizing the apoglucose oxidase as described in Japanese Patent Disclosure (166980/82).

The content of the target-binding substance and the apoenzyme may be appropriately selected so as to detect the signal from the labelled substance, depending on the reactivity of the target substance and the target-binding substance, the expected range of the level of the target substance in the sample, and on the sensitivity of the labelled substance. The selection of the content of these substances may easily be conducted by those skilled in the art without undue experiment and specific examples are presented in the Examples hereinbelow described.

In order to eliminate the non-specific reaction in the immunological reaction, the reaction layer also may contain a protein which does not participate or interfere the specific immunological reaction. Examples of such a protein include normal serum proteins of mammals, albumin and gelatin, as well as decomposition products thereof.

The thickness of the reaction layer may preferably be 10 - 200 $\mu$m, more preferably 30 - 180 $\mu$m.

c) Non-porous Layer

The non-porous layer comprises macromolecular substance in which the labelled substance is incorporated. The term "non-porous" herein means that the layer has substantially no communication holes.

The macromolecular substance may preferably be at least one hydrophilic colloid. Preferred examples of the hydrophilic colloid may include gelatin; gelatin derivatives such as phthalic acid-pretreated gelatin; synthetic polymers such as polyvinylalcohol, polyvinylpyrrolidone, polyvinylimidazole, polyacrylamide, sodium polyacrylate; cellulose derivatives such as hydroxyethyl cellulose and sodium carboxymethyl cellulose; and sodium alginate.

The non-porous layer may be obtained by applying the hydrophilic colloid or by forming a film with the hydrophilic colloid. The labelled substance may easily be incorporated by mixing a solution of the labelled substance in water or in an organic solvent with the hydrophilic colloid.

As mentioned above, the labelled substance contained in the non-porous layer is the target substance or an analogue thereof which is labelled with an appropriate marker. The analogue of the target substance should have an ability to specifically reacts with the above-described target-binding substance such as an antibody corresponding to the target substance. The labelled substance should have an ability to give a signal which is changed when the labelled substance is bound to the target-binding substance. To achieve this, the marker may preferably be a prosthetic group or a coenzyme of an enzyme, and the signal may be given by the association of the prosthetic group or the coenzyme with the above-described apoenzyme. The mechanism of generating the signal and method for measuring the same will be described in detail in the subsequent section. Preferred examples of the prosthetic group and the coenzyme may include, flavin adenine dinucleotide (FAD), nicotinamide adenine dinucleotide (NAD) and reduced form thereof (NADH), nicotinamide adenine dinucleotide phosphate (NADP) and reduced form thereof (NADPH), adenosine phosphate and flavin mononucleotide. Among these, FAD is most preferred. The target substance or an analogue thereof may be labelled with the marker by a conventional method disclosed in, for example, D.L. Morris et al., Analytical Chemistry, vol. 53, pp. 658-665 (1981); T.J. Richard et al., Clinical Chemistry, vol. 27, pp. 1499-1504 (1981); and Japanese Patent Disclosure (Kokai) Nos. 2996/80 and 46072/83.

The content of the labelled substance in the ncn-porous layer may be appropriately selected so as to detect the signal from the labelled substance, depending on the reactivity of the target substance and the target-binding substance, the expected range of the level of the target substance in the sample, and on the sensitivity of the labelled substance. The selection of the content of these substances may easily be conducted by those skilled in the art without undue experiment and specific examples are presented in the Examples hereinbelow described.

In order to retain the activity of the labelled substance, the non-porous layer may contain additives which are used for the preservation of proteins and enzymes. These substances are described in "Biochemistry Experiment 1, Chemistry of Proteins I", 1976, edited by Japan Biochemistry Association and published by Tokyo Kagaku Dojin), pp.66-67, "Experiment and Application, Affinity Chromatography" published by Kodansha Scientific, 1976 pp. 16 - 104, and Japanese Patent Disclosure (Kokai) No. 149927/85. Specific examples of these substances may include gelatin, decomposition products of gelatin, albumin, cyclodextrin, non-reduced saccharides (such as sucrose, trehalose), polyethyleneglycol, amino acids such as glycine, glycylglycine, various ions such as ammonium sulfate and sodium azide. The content of these substances in the non-porous layer may be appropriately selected.

The non-porous layer made of a hydrophilic colloid such as gelatin and gelatin derivative may also contain a hardening agent. The hardening agent widely used in the field of photograph may be employed. The hardening agents which may be employed are described in T. H. James ed., "The Theory of the Photographic Prorcess", 4th ed., pp. 77-87. That is, aldehydes, active olefins and active esters may be employed as the hardening agent. The content of the hardening agent in the non-porous layer is usually 10 mg/m$^2$ to 300 mg/m$^2$. By incorporating the hardening agent in the non-porous layer, the diffusion of the labelled substance into the reaction layer may be optionally controlled.

The thickness of the non-porous layer may preferably be 1 - 100 μm, more preferably 5 - 50 μm.

The above-described three layers may directly be laminated or coated. That is, the fibrous porous spreading layer a) may directly be laminated or coated on the non-fibrous porous layer b) and the non-fibrous porous layer b) may directly be laminated or coated on the non-porous layer c). However, if desired, conventional supplemental layers such as blood separation layer which is useful when the sample is whole blood, adhesive layer, protection layer and timing layer may be formed between the layers or on the spreading layer. Thus, the three layers a) - c) are not necessarily be laminated or coated directly.

Further, it is preferred that the three layers a) -c) and the supplemental layer, if any, are formed on a support film. Since in the preferred mode, as will be described later, the signal from the labelled substance is detected by colorimettric method and the coloring is preferably measured from the underside of the support film, the support film is preferably of light-transmitting nature. Preferred examples of the material for constituting the support film include polymers such as cellulose acetate, polyethylene terephthalate, polycarbonate and polyvinyl compounds such as polystyrene, as well as inorganic materials such as glass.

In operation, fluid sample is dropped on the fibrous spreading layer or on a supplemental layer such as a blood separation layer which is formed on the spreading layer. The fluid sample may be any solution or colloidal solution. Typically, the fluid sample may be a biological fluid such as blood, plasma, serum, cerebrospinal fluid, saliva, amniotic fluid, milk, urine, sweat, broth and the like.

Since the immunoassay element of the present invention has very high sensitivity, small amount of the sample may be assayed. That is, as small as 5 - 20 μl of the sample may be assayed with high precision.

Upon adding the fluid sample on the spreading layer or on the supplemental layer on the spreading layer, the fluid sample is passed through the spreading layer to reach the reaction layer. The sample fluid is uniformly spreaded while passing through the spreading layer.

Upon reaching the reaction layer, the target substance which may be contained in the sample specifically reacts with the target-binding substance.

Since the reaction layer is of porous structure, the sample fluid continues to move downward to reach the non-porous layer. Upon reaching the non-porous layer, since the non-porous layer is made of, for example, hydrophilic colloid, the labelled substance incorporated in the non-porous layer begins to diffuse to reach the reaction layer. Since the labelled substance also specifically binds to the target-binding substance contained in the reaction layer, the target substance in the sample and the labelled substance competitively reacts with the target-binding substance. As a result, part of the target-binding substance is bound to the target substance and part of the target-binding substance is bound to the labelled substance. The percentage of the target-binding substance bound to the target substance varies depending on the amount of the target substance in the sample. Similarly, part of the labelled substance is bound to the target-binding substance and the remainder remains free, and the percentage of the free labelled substance varies depending on the amount of the target substance contained in the sample. As mentioned above, the labelled substance gives a signal which is changed when it is bound to the target-binding substance. Therefore, a changed signal is given by the labelled substance, and the degree of the change of the signal is dependent on the percentage of the bound labelled substance, in turn, on the target substance in the sample. Therefore, by measuring the changed signal, the target substance in the sample can be quantified or detected. In the immunoassay element of the present invention, since the labelled substance is contained in the non-porous layer under the reaction layer, the reaction between the target substance and the target-binding substance occurs prior to the reaction between the labelled substance and the target binding substance. As mentioned above, in cases where the target substance is a low molecular substance which does not have immunogenecity, the antibody corresponding to the target substance is prepared by immunizing an animal with the target-carrier conjugate, and the thus prepared antibody usually has a greater affinity to the labelled substance than to the target substance. With the element of the present invention, since the reaction between the target substance and the antibody starts prior to the reaction between the labelled substance and the antibody, even if the antibody has greater affinity to the labelled substance than to the target substance, the target substance can be measured with high sensitivity.

As mentioned above, in a preferred mode of the present invention, the marker of the labelled substance is a prosthetic group or a coenzyme of an enzyme and the corresponding apoenzyme is contained in the reaction layer. In this case, the signal given by the labelled substance which is changed when the labelled substance is bound to the target-binding substance may preferably be coloring, fluorescence or luminescence. More particularly, the above-described preferred apoenzymes, when associated with the corresponding prosthetic group or the coenzyme, may be measured by utilizing a coloring reaction, fluorescent reaction or luminescent reaction. For example, in cases where the apoenzyme contained in the reaction layer is apoglucose oxidase, when associated with FAD, the glucose oxidase may be quantified by utilizing the coloring reaction between the hydrogen peroxide generated by the oxidation of the glucose which is the substrate of the glucose oxidase and a coloring reagent, which coloring reaction is catalyzed by peroxidase. Since the apoglucose oxidase is changed to the active glucose oxidase by the association with FAD, the amount of the active glucose oxidase varies depending on the amount of the free FAD-labelled substance. On the other hand, the intensity of the color varies depending on the amount of the hydrogen peroxide, and in turn on the amount of the active glucose oxidase. Therefore, the intensity of the color is dependent on the amount of the free labelled substance, and in turn on the amount of the target substance in the sample. Therefore, by measuring the intensity of the color generated by the coloring reaction, the target substance contained in the sample can be quantified.

The signal originated from the labelled substance may be measured by measuring the absorbance (colorimetric analysis), fluorescence or luminescence by a conventional method. Further, the signal may be measured by the so called rate assay in which the change of the signal with time is measured, or by the end point assay in which the signal at a certain time point is mesured. Among these, the absorbance method (colorimetric analysis) is most preferred. In the absorbance method, ultraviolet light, visible light and near infrared light can be employed. In cases where the sample is blood serum or blood plasma, in order to reduce the influence by the light absorption by the serum or the plasma, green light, red light or near infrared light is preferably employed.

As is apparent from the above description, in cases where the signal originated from the labelled substance is coloring, substrate of the enzyme and, in many cases, a coloring reagent, as well as an additional enzyme such as peroxidase required for the coloring reaction are necessary. The coloring reagents are well-known in the art and preferred examples of the coloring reagent include those colored by coupling reaction which are described in Japanese Patent Disclosure (Kokai) Nos. 94653/82, 94654/82, 94655/82 and 94656/82. Further, the following compounds may also preferably be used as the coloring reagent:

Aniline derivatives such as o-dianisidine and o- and p-toluidine; aromatic diamines such as o-phenylenediamine, N,N′-dimethyl-p-phenylenediamine, benzidine and 3,3′,5,5′-tetramethylbenzidine; phenol derivatives such as catechol, guaiacol, orcinol and pyrogallol; aromatic carboxylic acids such as salicylic acid, pyrocatechinic acid and gallic acid; leuco dyes such as leuco-malachite green and leuco-phenolphthalein; combination of 4- aminoantipyrine and phenol or naphthol derivative; combination of 3-methyl-2-benzothiazoline hydrazone and N,N′-dimethylaniline; combination of p-anisidine and 8-hydroxyquinoline; 2,2′-azinodi(3-ethyl-6-sulfobenzothiazoline); and 2-(4-hydroxy-3-methoxyphenyl)-4,5-bis(p-dimethoxyaminophenyl)imidazole.

The coloring reagent and the substrate of the enzyme, as well as the additional enzyme for the coloring reaction may be incorporated in the non-porous layer containing the labelled substance. Alternatively, these may be incorporated in a separate detection layer under the non-porous layer.

The detection layer may preferably be made of a macromolecular substance such as a hydrophilic colloid which may be used for forming the non-porous layer. The coloring reagent, the substrate and the additional enzyme may be incorporated therein by mixing the solution of these substances in water or in an organic solvent with the macromolecular substance.

In order to improve the physical properties of the detection layer such as degree of swelling and fusibility by heat, part of the macromolecular substance may be replaced with water-dispersible polymer such as macromolecular latex. Preferred examples of the macromolecular latex include those described in Japanese Patent Disclosure (Kokai) Nos. 116258/82 and 99752/83. Although 70% by weight of the hydrophilic colloid binder may be replaced with the macromolecular latex, it is preferred that the percentage of the hydrophilic colloid replaced with the macromolecular latex be not more than about 50% by weight.

The thickness of the detection layer may preferably be 3 - 50 μm, more preferably be 5 - 30 μm.

The detection layer, as well as other layers of the element may optionally contain other additives such as a buffering agent, preservative, surface active agent and mordant. The buffering agent which may be contained in the detection layer or other layers serves to make and keep the pH in the layer in the range preferred for the coloring reaction or enzyme reaction. The buffering agents which may be employed are described in "Manual of Chemistry, Basic Edition" , 1966, pp.1312-1320, edited by Japan Chemistry Association, published by Maruzen Co., Ltd., Tokyo; N. E. Good et al., Biochemistry, vol. 5, p.467 (1966); Imamura and Saito, "Domain of Chemistry", vol 30 (2), p. 79 (1976); W. J. Ferguson et al., Anal. Biochem., vol. 104, p.300 (1980). Specific examples of the buffering agents which may be employed may include salts of citric acid, boric acid, phosphoric acid, tris(hydroxymethyl)aminomethane, barbital, glycine and Good's buffering agent. The content of the buffering agent may be appropriately selected. These buffering agent may also be contained in the layers other than the detection layer. As the preservative and the hardening agent which may be incorporated in the detection layer, those described above in the description of the non-porous layer may be employed. Preferred examples of the surface active agent which may be

8

incorporated in the detection layer or in any other layers of the element include non-ionic and ionic surface active agents such as octylphenoxypolyethoxyethanol (commercially available from Rohm and Haas under the trade name of Triton X-100), nonylphenoxypolyglycidol (commercially available from Oline Co., Ltd. under the tradename of Surfactant 10G). The mordant which may be incorporated in the detection layer serves to intensively collect the detection product required for the measurement of the enzyme activity and, in the case where the detection product is a dye to promote the absorbance or to shift the wavelength. Preferred mordant may include polymers such as described in Japanese Patent Disclosure (Kokai) No. 87461/83 and latex of these polymers.

The immunoassay element of the present invention may preferably be manufactured as follows:

First of all, on a transparent support film, the detection layer and/or the non-porous layer is formed. This can be accomplished by applying an aqueous solution containing the hydrophilic binder and other components and drying the applied solution.

On the non-porous layer, the reaction layer is formed. In cases where the reaction layer is made of powder material such as microcrystalline cellulose or non-self adhering particles, an appropriate adhesive or binder is used to mutually adhering the powder material or the particles. The adhesive which may be employed is described in Japanese Patent Disclosure (Kokai) Nos. 53888/74, 90859/80 and 67860/82. In cases where the particles constituting the powder material have self-adhering property, the reaction layer may be prepared by a known method described in, for example, Japanese Patent Disclosure (Kokai) Nos. 101760/82, 101761/82 and 70163/83.

On the reaction layer, the spreading layer may be formed. This can be acchieved by applying a dispersion of fibers or fibers-particles mixture and drying the dispersion. Such a dispersion is known in the art and is described in, for example, Japanese Patent Disclosure (Kokai) Nos. 125847/82 and 197466/82. Dispersion may also be prepared by, as described in Japanese Patent Disclosure (173471/85) using a hydrophilic binder such as gelatin, polyvinylpyrrolidone and polyvinyl alcohol. The content of the hydrophilic binder may be selected from the wide range, and may preferably be 0.1 - 25% by weight, more preferably 1.0 - 20% by weight with respect to the weight of the fibers or the fibers-particles mixture. To prepare such a dispersion, various method may be employed individually or in combination. A useful method is to add a surface active agent to the medium of the dispersion. Preferred surface active agents are the same as the surface active agents which may be incorporated in the non-porous layer. The concentration of the surface active agent may be selected from a wide range, and may preferably be 20 - 0.005% by weight, more preferably 15 - 0.1% by weight with respect to the weight of the fibers or fibers-particles mixture. Further, ultrasonic treatment, physical mixing and/or agitation treatment may also be employed for the preparation of the dispersion. These methods are more useful if they are combined with the above-mentioned treatment with the surface active agent.

The invention will now be described by way of examples thereof. It should be noted that the examples are presented for the illustration purpose only and should not be interpreted any restrictive way.

In the examples, the FAD-labelled theophyllin and apoglucose oxidase were prepared in accordance with the method disclosed in USP4,238,565 or Japanese Patent Disclosure 46072/83.

### Example 1

#### 1-(1) Preparation of Anti-theophyllin Monoclonal Antibody

The anti-theophyllin monoclonal antibody was prepared as disclosed in Japanese Patent Disclosure 46072/83. That is, to 250 ml of anhydrous ethanol, 50 g of 6-chloro-1,3-dimethyluracil and 37 g of 6-aminocaproic acid were added and then 28 g of triethylamine was added. The resulting mixture was heated to reflux for 20 hours. After evaporating off the ethanol, uracil derivative in which (5-carboxypentyl)imino group was introduced was recrystallized from water. Thirty six grams of this product was suspended in 100 ml of water. To this suspension, 14 g of sodium nitrite dissolved in 50 ml of water was added, and the resulting mixture was allowed to react at $50^{\circ}$C for 1 hour. After cooling the mixture, precipitation was recovered by filtration.

In 400 ml of methanol, 32 g of this filtrate was dissolved and was reduced with hydrogen using platinum oxide as a catalyst. After removing the catalyst by filtration, the product was reacted with methyl formate to obtain a uracil derivative which was modified with formamide. Eighteen grams of the obtained uracil derivative was added to 1 liter of o-dichlorobenzene and the resulting mixture was heated to reflux for 4 hours under nitrogen atmosphere to obtain 9-(5-carboxypentyl)-1,3-dimethylxanthine. This product was bonded to bovine serum albumin by the method disclosed in USP4,238,565 or Japanese Patent Disclosure 46072/83 to obtain an immunogen.

Balb/c mice (female) of 6 weeks old were immunized with the thus obtained immunogen. That is, Balb/c mice were immunized by intraperitoneal injection of 200 μg of the immunogen in complete Freund's adjuvand. After two weeks, each mouse received an intraperitoneal injection of 150 μg of the immunogen in incomplete Freund's adjuvant. After two weeks from the second immunization, 100 μg of the immunogen dissolved in 0.15 M aqueous NaCl solution was intravenously administered to the mouse. After 3 days from the i.v. administration, spleen cells were removed from the mouse. The spleen cells ($4 \times 10^8$) were fused with azaguanine-resistant mouse myeloma cells X63-Ag8-6.5.3 ($1.2 \times 10^8$) and cell fusion operation was conducted according to the well-known polyethyleneglycol method using 50% polyethyleneglycol (Mw of 4000) solution. The fused cells were placed in wells of 96-well microtiter plates and were cultured in HAT medium (RPMI-1640 medium containing 0.4 μM of aminopterine, 16 μM of thymidine and 100 μM of hypoxanthine) containing 15% fetal calf serum, 50 units/ml of penicillin and 50 μg/ml of streptomycin. Half of the medium was exchanged with fresh medium every other day. After two weeks, the supernatant of the culture medium in each well was assayed by ELISA according to the conventional method and the positive cells were cloned by the well-known limiting dilution method to obtain hybridomas. The hybridomas were grown in abdominal cavity of a mouse to obtain mouse ascites fluid containing anti-theophyllin monoclonal antibody.

To 0.8 ml of the thus obtained mouse ascites, 200 mg of bovine serum albumin was added and the mixture was lyophilized. This lyophilized mixture was used for constructing a multilayered immunoassay element hereinbelow described.

1-(2) Preparation of Apo Glucose Oxidase

To 50 mg of lyophilized apoglucose oxidase, 50 ml of anti-glucose oxidase antibody obtained by immunizing a goat with glucose oxidase and 250 mg of bovine serum albumin were added and the resulting mixture was lyophilized. This lyophilized mixture was used for the construction of the multilayer immunoassay element hereinbelow described.

1-(3) Preparation of Immunoassay Element for Measuring Theophyllin

On a transparent polyethylene terephthalate film with a thickness of 180 μm, the coating solution 1 having the following composition was applied and dried to form a detection layer. The thickness of this detection layer after drying was 22 μm.

| Composition of Coating Solution 1 | |
|---|---|
| Deionized gelatin | 4.0 g |
| Triton X-100 (commercially available from Room and Haas) | 0.3 g |
| 1,7-dihydroxynaphthalene (commercially available from Aldrich) | 160 mg |
| Dimedone (1,1-dimethyl-3,5-cyclohexanedione, commercially available from Tokyo Kasei Co., Ltd.) | 50 mg |
| 4-aminoantipyrine hydrochloride (commercially available from Tokyo Kasei Co., Ltd.) | 230 mg |
| Glucose (commercially available from Tokyo Kasei Co., Ltd.) | 600 mg |
| Peroxidase (commercially available from Behringer) | 2500 U |
| 1,2-bis(vinylsulfonyl)ethane | 25 mg |
| Distilled water | 40.0 g |

On the detection layer, a coating solution 2 with a composition as hereinbelow described was applied to form a non-porous layer made of gelatin binder incorporating FAD-labelled theophyllin. The thickness of the layer after drying was 5 μm.

| Composition of Coating Solution 2 | |
|---|---|
| Deionized gelatin | 3.0 g |
| Triton X-100 | 0.3 g |
| FAD-labelled Theophyllin (aqueous solution of 10 μg/ml) | 80 μl |
| 1,2-bis(vinylsulfonyl)ethane | 18 mg |
| Distilled Water | 27.0 g |

A coating solution 3 containing uniformly dispersed anti-theophyllin antibody preparation including bovine serum albumin prepared in the above-described Example 1-(1) and apo glcose oxidase prepared in Example 1-(2), which has the composition as described later was applied on the non-porous layer and dried to form the non-fibrous porous reaction layer. The thickness of the layer after drying was 150 μm.

| Composition of Coating Solution 3 | |
|---|---|
| Triton X-100 | 0.4 g |
| Anti-theophyllin Antibody (Lyophilized) | 200 mg |
| Apoglucose oxidase containing anti-glucose oxidase (Lyophilized) | 380 mg |
| Polyvinylpyrrolidone (manufactured by Wako Pure Chemicals) | 1.8 g |
| Avicel (microcrystalline cellulose commercially available from Asahi Chemicals) | 11.0 g |
| n-butanol | 34.0 g |

Then a coating solution 4 with the composition described later was applied on the thus formed porous reaction layer and dried to form a fibrous porous spreading layer. The thickness of the spreading layer after drying was 200 μm.

| Composition of Coating Solution 4 | |
|---|---|
| Triton X-100 | 1.8 g |
| Polyvinylpyrrolidone | 1.8 g |
| Cellulose Powder D (Manufactured by Toyo Filter Paper Co., Ltd.) | 18.0 g |
| n-butanol | 68.0 g |

The thus prepared sample was cut into pieces sizing 1.5 cm x 1.5 cm to obtain an immunoassay element 1 of the present invention.

For comparison, an immunoassay element 2 was prepared in the same manner as the element 1 except that the FAD-labelled theophyllin was contained in the coating solution 3 for forming the non-fibrous porous reaction layer, not in the coating solution 2 for forming the non-porous layer.

1-(4) Assay of Theophyllin

Ten microliters of theophyllin solution (manufactured by Aldrich Co., Ltd.) in 50 mM citric acid-dipotassium hydrogen phosphate buffer (pH5.0) containing 5.0 wt% of bovine serum albumin, having a theophyllin level of 5 - 25 μg/ml was dropped onto the immunoassay element 1 or 2. The element was incubated in a sealed chamber at 37°C, and the density of the reflection light of 546 nm was measured every 30 seconds for 15 minutes. The reflection densities within 15 minutes are shown in Table 1. The values are indices wherein the value obtained for the sample containing no theophyllin was taken as 100.

Table 1

| Theophyllin Level (μg/ml) | Element 1 of Invention | Element 2 for Comparison |
|---|---|---|
| 0 | 100 | 100 |
| 5 | 120 | 105 |
| 10 | 138 | 113 |
| 15 | 157 | 120 |
| 20 | 172 | 131 |
| 25 | 198 | 138 |

As can be seen from Table 1, it was proved that with the multilayered immunoassay element 1 of the present invention in which the labelled substance is incorporated in the non-porous layer, theophyllin level can be measured with high sensitivity using a small amount of sample as small as 10 μl.

Example 2

2-(1) Preparation of Immunoassay Element for Measuring Theophyllin

On a transparent polyethylene terephthalate film with a thickness of 180 μm, the coating solution 5 with the composition described below was applied and dried to form the non-porous layer containing a labelled substance and a coloring reagent. The thickness of the non-porous layer after drying was 25 μm.

| Composition of Coating Solution 5 | |
|---|---|
| Deionized Gelatin | 4.5 g |
| Triton X-100 | 0.5 g |
| Glucose (manufactured by Tokyo Kasei) | 540 mg |
| 3,3′,5,5′-tetramethylbenzidine (manufactured by Dojin Chemicals Co., Ltd.) | 90 mg |
| Gantrez ES-225 (commercially available from GAF Co., Ltd.) (25 wt% solution in acetone) | 1.6 g |
| Peroxidase (manufactured by Behringer Co., Ltd.) | 3000 U |
| FAD-labelled Theophyllin (aqueous solution of 100 ug/ml) | 120 μl |
| 1.5M Citric Acid-Dipotassium Hydrogen Phosphate Buffer (pH 7.0) | 2.0 g |
| 1,2-bis(vinylsulfonyl)ethane | 30 mg |
| Distilled Water | 46.0 g |

On the thus formed non-porous layer, the non-fibrous porous reaction layer containing the anti-theophyllin antibody and apoglucose oxidase, as well as the fibrous porous spreading layer were formed as described in 1-(3), an the thus formed multilayered sample was cut into pieces sizing 1.5 cm x 1.5 cm to obtain immunoassay element 3 of the present invention.

2-(2) Measurement of Theophyllin Level

Theophyllin solution was prepared in the same manner as in 1-(4) and 10 μl thereof was dropped on the multilayered immunoassay element prepared in 2-(1). The element was incubated in a sealed chamber at 37° C, and the density of the reflection light of 650 nm was measured every 30 seconds for 15 minutes. The reflection densities within 15 minutes are shown in Table 2. The values are indices wherein the value obtained for the sample containing no theophyllin was taken as 100.

Table 2

| Theophyllin Level (μg/ml) | Element 3 of Invention |
|---|---|
| 0 | 100 |
| 5 | 122 |
| 10 | 141 |
| 15 | 161 |
| 20 | 184 |
| 25 | 205 |

As can be seen from Table 2, theophyllin can be quantified with high sensitivity with the immunoassay element in which the labelled substance is incorporated in the non-porous layer comprising the macromolecular substance.


Example 3


3-(1) Preparation of Immunoassay Element for Measuring Theophyllin

On a transparent polyethylene terephthalate film with a thickness of 180 μm, the coating solution 6 with the composition described below was applied and dried to form the non-porous layer containing a labelled substance and a coloring reagent. The thickness of the non-porous layer after drying was 20 μm.

| Composition of Coating solution 6 | |
|---|---|
| Deionized Gelatin | 4.0 g |
| Triton X-100 | 0.4 g |
| Glucose | 480 mg |
| 3,3',5,5,'-tetramethylbenzidine (manufactured by Dojin Chemicals Co., Ltd.) | 80 mg |
| Gantrez ES-225 (commercially available from GAF Co., Ltd.) (25 wt% solution in acetone) | 2.7 g |
| Peroxidase (manufactured by Behringer Co., Ltd.) | 2600 U |
| FAD-labelled Theophyllin (aqueous solution of 100 ug/ml) | 250 μl |
| 1.5M Citric Acid-Dipotassium Hydrogen Phosphate Buffer (pH 5.0) | 4.0 g |
| 1,2-bis(vinylsulfonyl)ethane | 27 mg |
| Distilled Water | 30.0 g |

A coating solution 7 containing uniformly dispersed anti-theophyllin antibody preparation including bovine serum albumin prepared in the above-described Example 1-(1) and apoglucose oxidase prepared in Example 1-(2), which has the composition as described later was applied on the detection layer (the non-porous layer) and dried to form the non-fibrous porous reaction layer. The thickness of the layer after drying was 100 μm.

| Composition of Coating Solution 7 | |
|---|---|
| Triton X-100 | 0.35 g |
| Anti-theophyllin Antibody (Lyophilized) | 250 mg |
| Apoglucose oxidase containing anti-glucose oxidase (Lyophilized) | 200 mg |
| Polyvinylpyrrolidone (manufactured by Wako Pure Chemicals) | 1.1 g |
| Avicel (microcrystalline cellulose commercially available from Asahi Chemicals) | 9.0 g |
| n-butanol | 32.0 g |

Then a coating solution 8 with the composition described later was applied on the thus formed porous reaction layer and dried to form a fibrous porous spreading layer. The thickness of the spreading layer after drying was 180 um.

13

| Composition of Coating Solution 8 | |
|---|---|
| Triton X-100 | 1.5 g |
| Polyvinylpyrrolidone | 0.8 g |
| Powder Filter Paper D (Manufactured by Toyo Filter Paper Co., Ltd.) | 11.0 g |
| n-butanol | 54.0 g |

The thus prepared sample was cut into pieces sizing 1.5 cm x 1.5 cm to obtain an immunoassay element 4 of the present invention.

For comparison, an immunoassay element 5 was prepared in the same manner as the element 4 except that the FAD-labelled theophyllin was contained in the coating solution 8 for forming the porous spreading layer, not in the coating solution 6 for forming the non-porous layer. That is, a coating solution 9 with the composition described below was used in place of the coating solution 8. By this operation, an immunoassay element 5 for comparison was obtained.

| Composition of Coating Solution 9 | |
|---|---|
| Triton X-100 | 1.5 g |
| Polyvinylpyrrolidone | 0.8 g |
| FAD-labelled Theophyllin (dimethylformamide solution of 100 μg/ml) | 170 μl |
| Powder Filter Paper D (Manufactured by Toyo Filter Paper Co., Ltd.) | 11.0 g |
| n-butanol | 54.0 g |

3-(2) Measurement of Theophyllin

To human serum collected from a normal human, theophyllin was added to a level of 0, 5, 15 or 25 μg/ml to prepare samples. Ten microliters of each sample was dropped onto the immunoassay element 4 or 5 obtained in 3-(1). The elements were incubated in a sealed chamber at 37°C, and the reflection density was measured at 650 nm from the side of the support film every 30 seconds for 7 minutes. This measurement was repeated ten times for each sample. The difference between the reflection density after 7 minutes and that after 3.5 minutes was calculated to conduct a rate assay. The results are shown in Table 3.

Table 3

| Element | Theophyllin (μg/ml) | Characteristics | | |
|---|---|---|---|---|
| | | $\bar{x}(\Delta Dr)$ | SD | CV (%) |
| Element 4 of the Invention | 0 | 0.086 | $2.1 \times 10^{-3}$ | 2.4 |
| | 5 | 0.124 | $2.2x \times 10^{-3}$ | 1.8 |
| | 15 | 0.197 | $3.0x \times 10^{-3}$ | 1.5 |
| | 25 | 0.265 | $5.3 \times 10^{-3}$ | 2.0 |
| Element 5 for Comparison | 0 | 0.091 | $9.8 \times 10^{-3}$ | 10.8 |
| | 5 | 0.111 | $11.7 \times 10^{-3}$ | 10.5 |
| | 15 | 0.135 | $11.1 \times 10^{-3}$ | 8.2 |
| | 25 | 0.152 | $14.3 \times 10^{-3}$ | 9.4 |

As can be seen from Table 3, theophyllin can be measured with higher sensitivity and with higher reproducibility by using the immunoassay element of the present invention than by using the element for comparison.

Although the invention was described based on the preferred embodiments thereof, it is apparent for those skilled in the art that various modification can be made within the spirit and scope of the present invention.

Claims

1. An immunoassay element for assaying a target substance in a fluid sample by utilizing homogeneous competitive reaction of the target substance and a labelled substance which is a target substance or an analogue thereof labelled with a marker, with a substance which specifically reacts with both the target substance and the labelled substance, which immunoassay element comprising, in the order mentioned from the top to the bottom, the layers of:

a) a fibrous porous spreading layer;

b) a non-fibrous porous reaction layer containing the substance which specifically reacts with both the target substance and the labelled substance; and

c) a non-porous layer containing a macromolecular substance in which the labelled substance is incorporated;

the labelled substance giving a signal which is changed when the labelled substance is bound to the substance which specifically reacts with both the target substance and the labelled substance.

2. The immunoassay element of claim 1, wherein the marker is a prosthetic group or a coenzyme of an enzyme.

3. The immunoassay element of claim 2, wherein the marker is selected from the group consisting of flavin adenine dinucleotide, nicotinamide adenine dinucleotide or a reduced form thereof, nicotinamide adenine dinucleotide phosphate or a reduced form thereof, adenosine phosphate and flavin mononucleotide.

4. The immunoassay element of claim 3, wherein the marker is flavin adenine dinucleotide.

5. The immunoassay element of claim 2, wherein the non-fibrous porous layer contains an apoenzyme of the prosthetic group or the coenzyme.

6. The immunoassay element of claim 2, wherein the apoenzyme is selected from the group consisting of the apoenzymes of glucose oxidase, glutathione reductase, lipoamide dehydrogenase, glucose-6-phosphate dehydrogenase, glutamate dehydrogenase, alcohol dehydrogenase and lactate dehydrogenase.

7. The immunoassay element of claim 6, wherein the apoenzyme is apoglucose oxidase and the apoglucose oxidase is stabilized by anti-glucose oxidase antibody.

8. The immunoassay element of claim 1, wherein the fibrous porous spreading layer is made of pulp, cellulose powder, or a fabric made of cotton, hemp, silk, wool, chitin, chitosan, cellulose ester, viscose rayon, copper ammonia rayon, polyamide, polyester, polyolefin, glass or asbestos fiber.

9. The immunoassay element of claim 1, wherein the fibrous porous spreading layer is made of an European paper, Japanese paper, filter paper, or a woven fabric, non-woven fabric or a synthesized paper made from a brush polymer.

10. The immunoassay element of claim 1, wherein the non-fibrous porous reaction layer has a porous structure made of at least one powder material with a particle size of 1 - 100 $\mu$m.

11. The immunoassay element of claim 10, wherein the powder material is selected from the group consisting of diatomateous earth, titanium dioxide, barium sulfate, zinc oxide, lead oxide, microcrystalline cellulose, siliceous sand, glass, silica gel, crosslinked dextran, crosslinked polyacrylamide, agarose, crosslinked agarose and self-binding particles.

12. The immunoassay element of claim 1, wherein the material constituting the macromolecular substance incorporating the labelled substance is at least one hydrophylic colloid with film-forming ability.

13. The immunoassay element of claim 12, wherein the hydrophilic colloid is selected from the group consisting of gelatin, gelatin derivatives, polyvinylalcohol, polyvinylpyrrolidone, polyvinylimidazole, polyacrylamide, sodium polyacrylate, polysaccharide of cellulose derivative and sodium alginate.

14. The immunoassay element of claim 13, wherein the hydrophilic colloid is at least one of gelatin and gelatin derivatives.

15. The immunoassay element of claim 1, further comprising a detection layer containing coloring reagents for detecting the signal from the labelled substance.

16. The immunoassay element of claim 1, wherein the non-porous layer further comprises a coloring reagent for detecting the signal from the labelled substance.

17. The immunoassay element of claim 1, wherein the thickness of the fibrous porous spreading layer is 50 -500 $\mu$m.

18. The immunoassay element of claim 17, wherein the thickness of the fibrous porous spreading layer is 100 -300 $\mu$m.

19. The immunoassay element of claim 1, wherein the thickness of the non-fibrous porous reaction layer is 10 - 200 $\mu$m.

20. The immunoassay element of claim 19, wherein the thickness of the non-fibrous porous reaction layer is 30 - 180 $\mu$m.

21. The immunoassay element of claim 1, wherein the thickness of the non-porous layer is 1 - 100 μm.

22. The immunoassay element of claim 21, wherein the thickness of the non-porous layer is 5 - 50 μm.

23. The immunoassay element of claim 1, further comprising a support film under the non-porous layer.

24. An immunoassay comprising the steps of:

providing the immunoassay element of claim 1;

dropping a sample fluid of 5 - 20 μl onto the fibrous porous spreading layer; and

measuring the signal from the labelled substance.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 051 213 (MILES LABORATORIES) * Page 12, line 30 - page 15, line 15; page 20, line 12 - page 22, line 23; page 24, lines 16-24; page 32, line 9 - page 36, line 34; page 73, line 1 - page 74, line 30 * --- | 1-9,12-24 | G 01 N 33/543 G 01 N 33/542 G 01 N 33/535 |
| X | EP-A-0 069 281 (MILES LABORATORIES) * Page 11, line 31 - page 13, line 8; page 17, line 25 - page 18, line 18; page 25, lines 17-26; page 27, lines 10-28; page 46, line 2 - page 49, line 6 * --- | 1-9,12-24 | |
| X | US-A-4 362 697 (D.L. TABB et al.) * Column 4, line 59 - column 5, line 32; column 6, lines 59-64; column 7, lines 20-68; column 9, lines 22-39; column 11, line 6 - column 15, line 21; column 20, lines 36-62 * --- | 1-9,12-24 | |
| Y | EP-A-0 061 071 (MILES LABORATORIES) * Page 3, line 29 - page 4, line 18; page 21, line 1 - page 23, line 13 * --- | 1-24 | TECHNICAL FIELDS SEARCHED (Int. Cl.4)  G 01 N |
| Y | CHEMICAL ABSTRACTS, vol. 109, 1988, page 332, abstract no. 34843m, Columbus, Ohio, US; & JP-A-62 249 062 (KONICA CO.) 30-10-1987 * Abstract * | 1-24 | |
| X | IDEM | 1,10,11 | |

The present search report has been drawn up for all claims

| Place of search | Date of completion of the search | Examiner |
|---|---|---|
| THE HAGUE | 30-05-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P0401)